# EUROPÄISCHE PATENTSCHRIFT

(11) **EP 1 764 354 B1**
(45) Veröffentlichungstag und Bekanntmachung des Hinweises auf die Patenterteilung: **02.11.2011**
(21) Anmeldenummer: 06017017.2
(22) Anmeldetag: 16.08.2006
(51) Int. Cl.: C07C 31/28, C07C 29/94, C07C 29/80, C07C 29/88, C07F 9/00, C23C 16/40

(54) **Verfahren zur Herstellung hochreiner Zirkonium-, Hafnium-, Tantal- und Niobalkoxide**
Process for preparing high purity zirconium-, hafnium-, tantalum-, and niobium-alkoxides
Procédé de préparation d'alkoxides de zirconium, de hafnium, de tantale et de nobium hautement purifiés

(30) Priorität: 27.08.2005 DE 102005040618; 03.11.2005 DE 102005052444
(43) Veröffentlichungstag der Anmeldung: 21.03.2007
(73) Patentinhaber: Heraeus Clevios GmbH, 63450 Hanau (DE)
(72) Erfinder: Reuter, Knud, 47800 Krefeld (DE); Zell, Friedrich, 79618 Rheinfelden (DE); Ebner, Martina, 79730 Murg (DE)
(74) Vertreter: Herzog, Martin

(56) Entgegenhaltungen:
- EP-A2- 0 251 432
- WO-A2-02/074473
- DATABASE CA [Online] CHEMICAL ABSTRACTS SERVICE, COLUMBUS, OHIO, US; KOKUNOV, YU. V. ET AL: "Structure of polymeric ethoxyfluoride complexes of niobium and tantalum in solution" XP002418802 gefunden im STN Database accession no. 85:85106 & KOORDINATSIONNAYA KHIMIYA , 2(5), 605-13 CODEN: KOKHDC; ISSN: 0132-344X, 1976,

## Beschreibung

Die Erfindung betrifft ein neues Verfahren zur Herstellung hochreiner Zirkonium-, Hafnium-, Tantal- und Niobalkoxide (-alkoholate) sowie neue Tantal- und Niobverbindungen und ein Verfahren zu deren Herstellung.

Zirkonium-, Hafnium-, Tantal- und Niobalkoxide (-alkoholate) lassen sich zur Abscheidung entsprechender Metalloxidschichten mittels Chemical Vapour Deposition (CVD) nutzen und sind daher wertvolle Ausgangsverbindungen zur Herstellung äußerst widerstandsfähiger Bauteile, die beispielsweise Verwendung in der Elektronikindustrie finden. Solche Metalloxidschichten lassen sich auch über die Hydrolyse nach der Sol-Gel-Methode aus den entsprechenden Zirkonium-, Hafnium-, Tantal- oder Niobalkoxiden herstellen. Die sohr hohe Dielektrizitätskonstante ermöglicht z.B. die Verwendung von Zirkonium-, Hafnium-, und Tantaloxidschichten in sog. DRAMs (Dynamic Random Access Read/Write Memories).

Allerdings besteht ein Problem für die Elektronikindustrie in extremen Anforderungen an die Reinheit der Ausgangsmaterialien für solche Schichten, d.h., den Alkoxiden. Es fehlt daher z.B. nicht an Beschreibungen spezieller Reinigungsverfahren für Niob- und Tantalalkoxide in der Patentliteratur. Die gebräuchlichste, technisch einfachste und wirtschaftlichste Herstellung der Zirkonium-, Hafnium-, Niob- und Tantalalkoxide geht von den entsprechenden Metallchloriden und Alkoholen aus. Einen umfassenden Überblick gibt das Werk "Alkoxo and Aryloxo Derivatives of Metalls" von D. C. Bradley, R. C. Mchrotra, I. Y. Rothwell und A. Singh, Academic Press, 2001. Eine typische Vorgehensweise ist z.B. in der DE 10113169 A1 beschrieben.

Die Herstellung aus den Metallchloriden führt zwangsläufig zu Chlorid als einer der abzutrennenden Hauptverunreinigungen in den Alkoxiden. Rohes Tantalethoxid vor der Destillation enthält deshalb um 500 - 1000 ppm oder mehr Cl. Beispielsweise enthalten Rohprodukte, die gemäß DE 10113169 A1 hergestellt wurden, typischerweise über 3000 ppm Cl. EP 0251432 offenbart ein zweistufiges Verfahren zur Herstellung von halogenarmen Metalloxiden wie Ta₂O₅ oder Nb₂O₅, bei welchem in einem ersten Verfahrensschritt ein Metallchlorid mit einem Überschuss an Alkohol und Ammoniak umgesetzt wird, bevor in einem zweiten Verfahrensschritt das entstandene Metallalkoxid hydrolisiert wird. Die erhaltene Metallalkoxide weisen einen von weniger als 10 ppm auf.

Daher ist die Entfernung von Chlorid auch der am häufigsten genannte Gegenstand der Erfindung in den o.g. Patenten zur Reinigung von Ta- und Nb-Alkoxiden. Dies liegt insbesondere daran, dass die Destillation alleine ein nur bedingt geeignetes Verfahren darstellt. Eine einfache Hochvakuumdestillation von rohem Tantalethoxid z.B. führt erfahrungsgemäß nur zu einer Absenkung des Cl-Gehalts auf etwa die Hälfte. Bessere Ergebnisse werden durch Destillationen über Füllkörperkolonnen erzielt. Wegen des hohen Siedepunkts der meisten Alkoxide, z.B. des Tantalethoxids, auch bei niedrigem Druck ist damit jedoch ein erheblicher Zeit- und Energieaufwand sowie eine technisch aufwändige Realisierung von einem Betriebsdruck < 1 mbar verbunden. Meist genügt die Trennwirkung einer einmaligen Destillation nicht aus, sodass eine wenig wirtschaftliche mehrfache Kolonnendestillation erforderlich wird. Auch bei der Entfernung von Cl aus Zirkonium- und Hafniumalkoxiden treten diese Schwierigkeiten auf.

Diese Problematik versuchen die Anmelder der JP 2002161059 A2 durch eine Nachbehandlung des rohen Tantalethoxids (z.B. mit 450 ppm Cl) mit ethanolischer Alkalimetallhydroxid-, speziell NaOH-Lösung zu lösen. Zwar wird der Cl-Gehalt nach diesem Verfahren in den gewünschten niedrigen Bereich abgesenkt, doch führt der Kontakt des Tantalethoxids mit Alkalimetallverbindungen bei solchen Operationen erfahrungsgemäß trotz Destillation zu unerwünschten erhöhten Alkalimetallgehalten im Produkt. Eine ähnliche Vorgehensweise wird in JP 06220069 A2 vorgeschlagen, wobei Alkalimetallhydride (z.B. LiH) oder Komplexvcrbindungen dieser Hydride benutzt werden. Auch hier besteht das Problem der zusätzlichen Kontamination der Tantalalkoxide mit Alkalimetallionen. Nicht anders ist die Methode zu schen, die in JP 06192148 A2 benutzt wird. Die dort eingesetzten Alkali- oder Erdalkalimetallalkoxide, z.B. Lithium- oder Natriumethoxid, vermindern den Cl-Cehalt ebenfalls in der gewünschten Weise, doch ist auch hier von erhöhten, unerwünschten Alkalüoncn-Konzentrationen im Produkt auszugeben. Beispielsweise steigt typischerweise der Na-Wert durch Zusatz von Natriumethoxid zum Tantalethoxid trotz anschließender Destillation von < 1 ppm Na auf 2 - 4 ppm Na. Schlieblich wird auch in JP 10036299 A2 eine Nachbehandlung mit Alkali- oder Erdalkalimetallverbindungen angegeben, wobei hier Carbonate benutzt werden. Die Effekte auf die Alkali- oder Erdalkalimetall-Kontaminationen der von Cl weitgehend befreiten Produkte sind auch hier im gleichen Sinne wie bei den o.g. Patentanmeldungen nachteilig. Das in dieser Anmeldung ebenfalls vorgeschlagene Silbercarbonat ist schon aus wirtschaftlichen Gründen unvorteilhaft.

In allen genannten Anmeldungen werden basische Verbindungen der Alkali- und Erdalkalimetalle zur Reinigung benutzt. Dies hat offensichtlich seinen Grund in der Tatsache, dass bei den technisch üblichen Herstellungsmethoden der Tantal- und Niobalkoxide die Verwendung von Ammoniak als Hilfsbase zur Umsetzung der Metallpentahalogenide mit Alkoholen keine Produkte ergibt bzw. zulässt, die ohne zusätzliche Nachbehandlungs- und Aufreinigungsschritte incl. aufwändiger Destillationen weniger als z.B. 100 ppm Cl enthalten. Die Lehre des Standes der Technik ist somit, dass mit Ammoniak als Hilfsbase bei der Umsetzung von Tantal- bzw. Niobchlorid mit Alkoholen Rohprodukte erhalten werden, die vor der weiteren Reinigung durch Destillation stets mehr als 100 ppm, meist ein Vielfaches davon, an Cl enthalten.

Aufgabe der vorliegenden Erfindung ist daher, ein Verfahren bereitzustellen, das es ohne den Einsatz von Alkali- oder Erdalkalimetallverbindungen erlaubt, Rohprodukte an Zirkonium-, Hafnium-, Tantal- und Niobalkoxiden herzustellen, die weniger als 200, insbesondere weniger als 100, bevorzugt weniger als 50 ppm Cl enthalten, bevor sie durch Destillation weiter gereinigt werden. Eine solches Verfahren ermöglicht es demzufolge, Cl-arme, weitestgehend alkalifreie Produkte und einen niedrigen Destillationsaufwand miteinander zu verbinden, wie dies bisher technisch nicht möglich war.

Überraschend wurde gefunden, dass es unter geeigneten, speziell ausgewählten Bedingungen unter Verwendung von Alkoholen und Ammoniak entgegen der Lehre des Standes der Technik möglich ist, Zirkonium-, Hafnium-, Tantal- und Niobalkoxide mit Halogengehalten, insbesondere Cl-Gehalten, von weniger als 200, insbesondere weniger als 100, bevorzugt weniger als 50 ppm vor ihrer nachfolgenden Destillation bereitzustellen.

Gegenstand der Erfindung ist ein Verfahren zur Herstellung von hochreinen Metallalkoxiden M(OR)ₓ, in denen M für Nb, Ta, Zr oder Hf, bevorzugt für Nb oder Ta steht, x im Falle von M = Nb oder Ta für 5 und im Falle von M = Zr oder Hf für 4 steht und R unabhängig voneinander gleiche oder verschiedene C₁-C₁₂-Alkylreste bedeutet, dadurch gekennzeichnet, dass
- rohe Metallalkoxide M(OR)ₓ mit einem Halogengehalt, insbesondere Cl-Gehalt > 100 ppm, gegebenenfalls > 200 ppm, die als Verunreinigung mindestens 0,05 Gew.-%, bevorzugt 0,1 bis 10,0 Gew.-% ein- oder mehrkerniger halogenhaltiger Metallalkoxide enthalten,
- mit maximal 30 Gew.-%, bevorzugt 4 bis 12 Gew.-%, bezogen auf die Gesamtmenge des rohen Alkoxids, eines Alkohols ROH, wobei R für einen C₁-C₁₂-Alkylrest steht, gemischt werden und
- anschließend oder gleichzeitig (z.B. nach vorherigem Lösen im Alkohol ROH) ein Überschuss, bezogen auf die Menge an ein- oder mehrkernigen halogenhaltigen Metallalkoxiden, bevorzugt von 0,1 bis 5,0 Gew.-%, bezogen auf die Gesamtmenge des rohen Alkoxids, Ammoniak zudosiert wird.

Bevorzugt stehen im Verfahren der Rest R für C₁-C₅-Alkyl und Hal für Cl. Besonders bevorzugt stehen im Verfahren der Rest R für C₁-C₅-Alkyl, Hal für Cl und M für Ta. Ganz besonders bevorzugt ist ein Verfahren indem in der Verbindung nach Formel (I) M für Ta, R für Ethyl, Hal für Cl stehen.

Wesentlich für diese Reinigungsoperation bei den Tantal- und Niobalkoxiden ist die Herkunft des Hauptteils analytisch nachgewiesenen Halogenids, vorzugsweise Cl, aus dem Vorliegen folgender, in der Literatur bisher nicht beschriebener Verbindung im rohen Produktgemisch

M₂(OR)₉Hal (I),

Dabei bedeutet M Niob oder Tantal, R eine C₁- bis C₁₂-Alkylgruppe, und Hal ein Halogen aus der Gruppe F, Cl, Br, I, vorzugsweise Cl.

In ähnlicher Weise ist die Herkunft des Hauptteils analytisch nachgewiesenen Halogenids, vorzugsweise Cl, in rohen Zirkonium- und Hafniumalkoxiden auf das Vorliegen folgender gegebenenfalls komplexer Verbindungen im rohen Produktgemisch

Mₚ(OR)_{4p-q}-Hal_{q} (II)

mit q = 1, 2, 3 oder 4, hauptsächlich 1 oder 2 zurückzuführen, wobei wegen der molekularen Komplexität der Zirkonium- und Hafniumalkoxide, außer gegebenenfalls bei bestimmten verzweigten, vorzugsweise tertären Alkoxiden, p > 1, und zwar 2, 3 oder 4, hauptsächlich 3 oder 4 ist. Zur Komplexität, d. h. dem Vorliegen oligomerer Cluster, in Zr- und Hf-Alkoxiden siehe z.B. "Alkoxo and Aryloxo Derivatives of Metals" von D. C. Bradley, R. C. Mehrotra, I. P. Rothwell und A. Singh, Academic Press, 2001. Dabei bedeutet M Zirkonium oder Hafnium, R eine C₁- bis C₁₂-Alkylgruppe, und Hal ein Halogen aus der Gruppe F, Cl, Br, I, vorzugsweise Cl.

Somit handelt es sich bei den ein- oder mehrkernigen halogenhaltigen Metallalkoxiden, die mittels des erfindungsgemäßen Verfahrens aus den rohen Alkoxiden entfernt werden, im Falle von M = Nb oder Ta im Wesentlichen um die zweikernige halogenhaltige Verbindung der Formel M₂(OR)₉Hal und im Falle von M = Zr oder Hf im Wesentlichen um eine oder mehrere mehrkernige komplexe halogenhaltige Verbindungen der Formel Mₚ(OR)_{4p-q}Hal_{q}.

Insbesondere im Falle bestimmter verzweigter, insbesondere tertiärer Alkylreste R ist es sowohl bei Zirkonium- und Hafniumalkoxiden als auch bei Tantal- und Niobalkoxiden auch möglich, dass einkernige halogenhaltige Metallalkoxide, d.h. Verbindungen der allgemeinen Formel (II), worin p für 1 steht, als Verunreinigungen im rohen Produktgemisch vorliegen.

**C₁-C₁₂-Alkyl** steht im Rahmen der Erfindung beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, n-Heptyl, n-Octyl, n-Nonyl, n-Decyl und n-Dodecyl.

Insbesondere sind bevorzugte Reste C₁-C₅-Alkyl solche aus der Gruppe Methyl, Ethyl, n-Propyl, iso-Propyl, n-Butyl, sec.-Butyl, n-Pentyl; besonders bevorzugt sind Ethyl, n-Propyl, n-Butyl, n-Pentyl.

Geeignete, bevorzugt zu reinigende Verbindungen sind z.B. Tantalmethoxid, Tantalethoxid, Tantal-n-propoxid, Tantal-2-propoxid, Tantal-n-butoxid, Tantal-2-butoxid (= Tantal-sec-butoxid), Tantal-iso-butoxid (= Tantal-2-methyl-1-propoxid), Tantal-tert-butoxid, Tantal-n-pentoxid und die entsprechenden Niobverbindungen. Besonders bevorzugt sind Tantalethoxid, Tantal-n-propoxid, Tantal-n-butoxid und Tantal-n-pentoxid.

Es ist im neuen Verfahren möglich, neben den im Gemisch vorliegenden, durch M₂(OR)₉Hal oder Mₚ(OR)_{4p-q}Hal_{q} verunreinigten Verbindungen M(OR)ₓ und ROH auch zusätzlich inerte Lösungsmittel, d.h. nicht mit den Metallverbindungen reagierende Lösungsmittel, zu verwenden. Solche Lösungsmittel sind zum Beispiel aliphatische lineare, verzweigte oder cyclische Kohlenwasserstoffe wie n-Pentan, n-Hexan, n-Heptan, Isooctan, Cyclohexan oder aromatische Kohlenwasserstoffe wie Toluol oder Xylol, oder Gemische solcher Lösungsmittel. Der Gewichtsanteil dieser zusätzlich verwendeten Lösungsmittel überschreitet typischerweise nicht die Gewichtsmenge an Metallverbindungen.

Die Syntheseschritte, die zu den o.g. rohen Metalloxiden geführt haben, sind dabei für das erfindungsgemäße Reinigungsverfahren belanglos, wobei jedoch stets eine Synthese aus Metallhalogeniden MHal₅ erfolgt sein muss. Solche Synthesen sind in der Literatur und Patentliteratur vielfach beschrieben, siehe z.B. in "Alkoxo and Aryloxo Derivatives of Metals" von D. C. Bradley, R. C. Mehrotra, I. P. Rothwell und A. Singh, Academic Press, 2001. Das erfindungsgemäße Verfahren betrifft nicht die Reinigung roher Metallalkoxide, die nach anderen Verfahren als aus MHal₅ und ROH, z.B. aus Metallamiden oder -amidimiden und ROH, oder z.B. durch Elektrolyse der Metalle M in Alkoholen ROH hergestellt worden sind.

Die Aufarbeitung der gemäß obiger Beschreibung mit Ammoniak/ROH umgesetzten Alkoxide erfolgt durch Abfiltration des entstandenen Ammoniumhalogenids und destillative Entfernung aller Lösungsmittel, z.B. unter vermindertem Druck. Es ist dabei vorteilhaft, aber nicht zwingend, vor der Filtration den eingesetzten Alkohol ROH durch Destillation - bevorzugt unter vermindertem Druck - zu entfernen. Sofern diese Entfernung durchgeführt wird, ist es wichtig, eine niedrigere Temperatur, bevorzugt < 40°C, zu wählen. Bei höheren Temperaturen, insbesondere bei 80 - 100°C und höher, tritt - im Folgenden beispielhaft für die Tantal- und Niobalkoxide detailliert aufgeführt - bei Anwesenheit von Ammoniumhalogenid durch die im Gleichgewicht vorliegende HHal-Konzentration eine Reaktion des Metallalkoxids gemäß folgender Reaktionsgleichung ein, die der Klarheit halber für die dimeren Alkoxide formuliert wurde, wie sie tatsächlich meist anstelle der vereinfachend so bezeichneten "Pentaalkoxide" M(OR)₅ vorliegen:

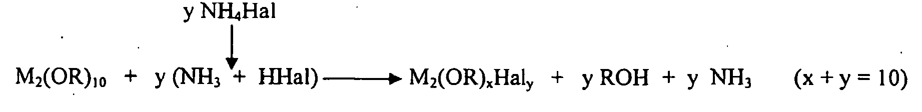

Für nicht dimerisierende Metallalkoxide, bei denen tatsächlich Moleküle M(OR)₅ vorliegen, ist die Reaktionsgleichung sinngemäß analog zu formulieren:

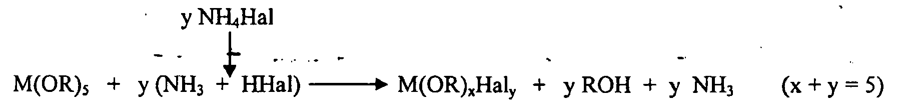

Durch diese Reaktion wird bei erhöhten Temperaturen der Halogenanteil im zu reinigenden Produkt wieder erhöht, sodass der Erfolg der Reinigungsoperation mit NH₃/ROH wieder zunichte gemacht würde.

Für die Zirkonium- und Hafniumalkoxide M(OR)₄ können sinngemäß ähnliche Reaktionen formuliert werden.

Vorteilhaft ist auch eine bevorzugte Variante, in der das nach der Reaktion mit NH₃/ROH erhaltene Gemisch vor oder nach der destillativen Entfernung von ROH mit einem aliphatischen Kohlenwasserstoff (KW) wie n-Pentan, n-Hexan, n-Heptan, Isooctan, Cyclohexan oder einem aromatischen KW, z.B. Toluol oder Xylol, versetzt und anschließend filtriert wird. Dabei kann die Menge an zugesetztem KW breit variiert werden; besonders vorteilhaft sind Gewichtsmengen von 20 bis 200 %, bezogen auf die Menge an rohem Alkoxid.

Es gibt also zu dem neuen Verfahren verschiedene bevorzugte Varianten der Aufarbeitung nach der Umsetzung des rohen Alkoxids mit NH₃/ROH:
1) Filtration, dann destillative Entfernung von ROH.
2) Destillative Entfernung von ROH, dann Filtration.
3) Filtration, destillative Entfernung von ROH, Zusatz von KW, Filtration
4) Destillative Entfernung von ROH, Zusatz von KW, Filtration.
5) Zusatz von KW, Filtration, destillative Entfernung von KW/ROH.

Es sind noch weitere Abfolgen aus Filtration/Entfernung von ROH/Zusatz von KW denkbar und durchführbar. Besonders vorteilhaft für die Reinheit des Produkts sind die Varianten 3) und 4).

Im Anschluss an die erfindungsgemäße Reinigung der rohen Alkoxide schließt sich üblicherweise eine Destillation an. Es ist ein besonderer Vorteil der Erfindung, dass diese Destillation nunmehr auch ohne große Trennleistung, d.h. auch ohne Füllkörperkolonne oder eine Destillationsapparatur vergleichbarer Trennleistung, und nur einmalig durchgeführt werden muss, um ein Alkoxid mit weniger als 200, insbesondere weniger als 100, bevorzugt unter 50 ppm Hal zu erhalten. Somit erhöht die Erfindung durch Zeit-, Energie- und apparative Einsparungen die Wirtschaftlichkeit der Metallalkoxid-Herstellung erheblich.

Gegenstand der Erfindung sind ebenfalls die bisher in der Literatur nicht beschriebenen Verbindungen M₂(OR)₉Hal sowie Gemische aus M(OR)₅ und mindestens 0,05 Gew.-% M₂(OR)₉Hal, bevorzugt 0,1 bis 10 Gew.-% M₂(OR)₉Hal, worin M für Ta oder Nb steht.

Bevorzugt sind Verbindungen beziehungsweise die entsprechenden Gemische mit R = C₁-C₅-Alkyl und Hal = Cl. Besonders bevorzugt sind Tantalverbindungen und ihre Gemische mit R = C₁-C₅-Alkyl und Hal = Cl. Ganz besonders bevorzugt ist M = Ta, R = Ethyl, Hal = Cl.

Die Herstellung der Verbindungen M₂(OR)₉Hal kann beispielsweise dadurch erfolgen , dass man Metallalkoxide M(OR)₅ bzw. M₂(OR)₁₀ im geeigneten stöchiometrischen Verhältnis mit Halogeniden MHal₅ umsetzt ("Komproportionierung"). Diese Komproportionierungsreaktion kann mit einem Lösungsmittel, bevorzugt ohne Lösungsmittel, bei erhöhter Temperatur, bevorzugt 40 bis 120°C, durchgeführt werden. Alternativ bietet sich für die Herstellung dieser Verbindungen die Umsetzung der Metallalkoxide mit Acetylhalogeniden im Molverhältnis 2 : 1 an, z.B. im Falle von Hal = Cl mit Acetylchlorid.

Diese neuen Verbindungen können beispielsweise als Edukte für Folgeverbindungen, die durch Substitution des Hal gegen geeignete Nucleophile, wie z.B. gegebenenfalls substituierte Aminogruppen, Alkylgruppen, Alkylthio- oder andere Alkoxygruppen entstehen, genutzt werden.

Die Komproportionierung eignet sich auch zur Herstellung höher halogenierter, gemischter Metallalkoxid-halogenide, wie z.B. M(OR)₄Hal bzw. M₂(OR)₈Hal₂.

Die folgenden Beispiele dienen der beispielhaften Erläuterung der Erfindung und sind nicht als Beschränkung aufzufassen.

### Beispiele

### Beispiel 1 Reinigung von rohem Tantalethoxid

Einwaagen:

| | |
|---|---|
| 2992 g | rohes, undestilliertes Tantalethoxid, hergestellt gemäß DE 10113169 A1 |
| 299 g | Ethanol abs. |
| 15,55 g | Ammoniak gasförmig (gasf.) und wasserfrei (wssfr.) |

Tantalethoxid (3500 ppm Cl) und Ethanol wurden vorgelegt; bei RT ohne Kühlung wurde unter Rühren in 2 h Ammoniak eingeleitet (exotherm bis ca. 30°C). Der Ansatz wurde weitere 2 h bei RT nachgerührt. Nach Stehen über Nacht wurde bei maximal 40°C Ethanol im Wasserstrahlvakuum, zum Schluss im Hochvakuum < 1 mbar abgezogen. Danach wurden 1500 ml Hexan zugesetzt und das Gemisch über ein Faltenfilter filtriert. Hexan wurde danach bei 16 mbar/80°C, zum Schluss bei < 1 mbar/80°C entfernt.

Cl (colorimetrisch) 25 ppm.

### Beispiel 2 Herstellung von Tantalethoxid und Reinigung

53,8 g (150 mmol) Tantalchlorid wurden in 30 ml trockenem Heptan suspendiert. Hierzu wurden innerhalb von ½ h 500 ml (853 mmol) abs. Ethanol zudosiert, wobei durch Kühlung mit Wasser/Eis die Innentemperatur unter 30°C gehalten und in der Anfangsphase zur Minderung der Exothermie nur langsam zugetropft wurde. Es wurde ½ h bei 25°C nachgerührt.

Danach wurden bei maximal 30°C (Kühlung mit Wasser/Eis) 24,94 g (1,464 mol) Ammoniak innerhalb von 1,5 h eingeleitet. Nach Stehen über Nacht wurde vom Ammoniumchlorid abfiltriert und Ethanol bei 20 mbar, zum Schluss im Hochvakuum bei < 1 mbar abdestilliert.

Zu 42,9 g des erhaltenen rohen Ethoxids wurden 4,3 g abs. Ethanol gegeben; anschließend wurden bei RT in 1 h 2,07 g (121 mmol) Ammoniak eingeleitet. Nach 1,5 h Nachrühren bei 23°C wurde bei < 40°C Ethanol abgezogen, 40 ml Hexan zugesetzt und filtriert. Anschließend wurde das Hexan bei 20 mbar/< 40°C abdestilliert. Cl (colorimetrisch): 33 ppm.

### Beispiel 3 Herstellung von Niobethoxid und Reinigung

40,5 g (150 mmol) Niobchlorid wurden in 30 ml trockenem Heptan suspendiert. Hierzu wurden innerhalb von ½ h 550 g abs. Ethanol zudosiert, wobei durch Kühlung mit Wasser/Eis die Innentemperatur unter 40°C gehalten und in der Anfangsphase zur Minderung der Exothermie nur langsam zugetropft wurde. Es wurde ½ h bei 25°C nachgerührt.

Danach wurden bei maximal 35°C (Kühlung mit Wasser/Eis) 16,61 g (975 mmol) Ammoniak innerhalb von 1,5 h eingeleitet. Danach wurde vom Ammoniumchlorid abfiltriert und Ethanol bei 20 mbar, zum Schluss im Hochvakuum bei < 1 mbar abdestilliert.

Zum erhaltenen rohen Ethoxid wurden 3,5 g abs. Ethanol gegeben; anschließend wurden bei 23°C in ½ h 1,69 g (992 mmol) Ammoniak eingeleitet. Nach 2 h Nachrühren bei 23°C wurden 40 ml Hexan zugesetzt und das Gemisch filtriert; Nachwaschen des abfiltrierten Ammoniumchlorids mit Hexan. Die Hexan-Lösungen wurden aufdestilliert; das nach Abdestillation des Hexans erhaltene Niobethoxid enthielt ca. 40 ppm Cl. Nach Destillation bei 160°/0,49 mbar wurden 35,9 g = 75 % d.Th. Niobethoxid erhalten, Cl (colorimetrisch) 20 ppm.

### Beispiel 4 Herstellung von Ta₂(OEt)₉Cl aus Tantalethoxid und Tantalchlorid

3,58 g (10 mmol) Tantalchlorid und 36,56 g Tantalethoxid (90 mmol) wurden gemischt und unter Rühren 7 h auf 80°C erhitzt. Nach Abkühlen wurde ein flüssiges Produkt über wenigen festen Anteilen erhalten. Die flüssige Phase wurde abdekantiert (ca. 3 g) und bestand aus Ta₂(OEt)₉Cl.

¹H-NMR (C₆D₆, 400 MHz, δ gegen TMS): 1,24 (t, J= 6,85 Hz); 1,32 (t, J= 6,85 Hz); 1,44 (t, *J* = 6,85 Hz); 4,62 (m, br); 4,85 (m, br).

| | | | |
|---|---|---|---|
| Elementaranalyse %: | C 26,9; | H 5,65; | Cl 4,42. |
| C₁₈H₄₅ClO₉Ta₂: | C 27,1; | H 5,9 ; | Cl4,32. |

### Beispiel 5 Herstellung von Ta₂(OEt)₉Cl aus Tantalethoxid und Acetylchlorid

40,62 g (100 mmol) Tantalethoxid wurden in 100 ml trockenem Toluol vorgelegt. Dazu wurden 3,92 g Acetylchlorid (50 mmol) getropft und das Gemisch 1 h zum Rückfluss erhitzt. Anschließend würden alle flüchtigen Bestandteile bis 30°C/0,5 mbar abdestilliert. Der flüssige Rückstand wurde von geringen festen Anteilen abdekantiert und bestand aus Ta₂(OEt)₉Cl.

¹H-NMR (C₆D₆, 400 MHz, δ gegen TMS, ppm): 1,24 (t, *J* = 6,85 Hz); 1,31 (t, *J* = 6,85 Hz); 1,44 (t, *J* = 6,85 Hz); 4,62 (m, br); 4,84 (m, br).

### Beispiel 6

Herstellung von Ta(OEt)₄Cl gemäß Lit. Kapoor, R. N.; Prakash, Sarla; Kapoor, P. N. Indian Journal of Chemistry (1967), (5 (9), 442-3 (Vergleichsbeispiel zum Beleg der unterschiedlichen Identität zum Ta₂(OEt)₉Cl, nicht erfindungsgemäß)

20,3 g (50 mmol) Tantalethoxid wurden in 100 ml trockenem Toluol vorgelegt. Dazu wurden 3,92 g Acetylchlorid (50 mmol) getropft und das Gemisch 1 h zum Rückfluss erhitzt. Anschließend wurden alle flüchtigen Bestandteile bis 30°C/0,5 mbar abdestilliert. Weißer, wachsartiger Rückstand: Ta(OEt)₄Cl.

Schmp. 61 - 65°C

¹H-NMR (C₆D₆, 400 MHz, δ gegen TMS, ppm): 1,21 (12H, t, *J* = 6,85 Hz); 1,30 (6H, t, br); 1,48 (6H, t, *J* = 6,85-Hz); 4,59 (4H, m, br); 4,74 (4H, m, *J* = 6,85 Hz); 4,85 (8H, m, br).

| | | | |
|---|---|---|---|
| Elementaranalyse %: | C 22,7; | H 4,53; | Cl 9,15; |
| C₈H₂₀ClO₄Ta: | C 24,2; | H 5,08; | Cl 8,94. |

### Beispiel 7

Herstellung von Ta(OEt)₄Cl durch Komproportionierung (zum Beleg der unterschiedlichen Identität zum Ta₂(OEt)₉Cl, nicht erfindungsgemäß)

11,02 g (30,8 mmol) Tantalchlorid und 50,00 g Tantalethoxid (123 mmol) wurden gemischt und unter Rühren 6 h auf 80°C erhitzt. Nach Abkühlen wurde ein einheitliches weißes, wachsartiges Produkt erhalten.

Schmp. 65°C

¹H-NMR (C₆D₆, 400 MHz, δ gegen TMS, ppm): 1,21 (12H, t, *J* = 6,85 Hz); 1,30 (6H, t, br); 1,48 (6H, t, J= 6,85 Hz); 4,59 (4H, m, br); 4,74 (4H, m, J= 6,85 Hz); 4,85 (8H, m, br).

### Beispiel 8 Reinigung von rohem Hafniumethoxid

Einwaagen:

| | |
|---|---|
| 25 g | rohes, undestilliertes Hafniumethoxid, hergestellt gemäß DE 10113169 A1 aus HfCl₄ |
| 2 g | Ethanol abs. |
| 38 ml | Hexan |
| 1,5 g | Ammoniak gasförmig und wasserfrei |

Hafniumethoxid (> 1000 ppm Cl), Ethanol und Hexan wurden vorgelegt; bei RT ohne Kühlung wurde unter Rühren in 35 min Ammoniak eingeleitet. Der Ansatz wurde weitere 2¼ h bei RT nachgerührt. Danach wurde bei maximal 40°C etwa 7,5 g Ethanol/Hexan-Gemisch im Wasserstrahlvakuum abgezogen. Danach wurden 19 ml Hexan zugesetzt und das Gemisch über ein Faltenfilter filtriert. Hexan wurde danach bei 16 mbar/80°C, zum Schluss bei < 1 mbar/70°C entfernt.

Cl (colorimetrisch) 128 ppm.

## Patentansprüche

1. Verfahren zur Herstellung von hochreinen Metallalkoxiden M(OR)ₓ, in denen M für Nb, Ta, Zr oder Hf, x im Falle von M = Nb oder Ta für 5 und im Falle von M = Zr oder Hf für 4 und R unabhängig voneinander für gleiche oder verschiedene C₁-C₁₂₋Allylreste stehen, **dadurch gekennzeichnet, dass**
• ein Rohprodukt des Metallalkoxids M(UR)ₓ mit einem Halogengehalt > 200ppm, die als Verunreinigung mindestens 0,05 Gew.-% ein- oder mehrkerniger halogenhaltiger Metallalkoxide enthalten,
• mit maximal 30 Gew.-% bezogen auf die Gesamtmenge des rohen Alkoxids, eines Alkohols ROH, wobei R für einen C₁-C₁₂-Alkylrest steht, gemischt werden und
• anschließend oder gleichzeitig ein Überschuss, bezogen auf die Menge an ein- oder mehrkernigen halogenhaltigen Metallalkoxiden, Ammoniak zudosiert wird.

2. Verfahren gemäß Anspruch 1, **dadurch gekennzeichnet, dass** M für Ta oder Nb steht und das als Verunreinigung enthaltene ein- oder mehrkernige halogenhaltige Metallalkoxid die Verbindung M₂(OR)₉Hal ist, worin Hal für F, Cl, Br oder I steht und R die in Anspruch I genannte Bedeutung hat.

3. Verfahren gemäß Anspruch 1, **dadurch gekennzeichnet, dass** M für Zr oder Hf steht und das oder die als Verunreinigung enthaltenen ein- oder mehrkemige(n) halogenhaltige(n) Metallalkoxid(e) wenigstens eine Verbindung Mₚ(OR)_{4p-q}Hal_{q} ist, worin Hal für F, Cl, Br oder 1, q für 1, 2, 3 oder 4, p für 2, 3 oder 4 stehen und R die in Anspruch 1 genannte Bedeutung hat.

4. Verfahren gemäß wenigstens einem der Ansprüche 1 bis 3, **dadurch gekennzeichnet, dass** der Rest R für C₁-C₅-Alkyl und Hal für Cl stehen.

5. Verfahren gemäß wenigstens einem der Ansprüche 1 bis 4, **dadurch gekennzeichnet, dass** der Rest OR eine Ethoxygruppe ist und M für Ta steht.

6. Verfahren gemäß wenigstens einem der Ansprüche 1 bis 5. **dadurch gekennzeichnet, dass** das Rohprodukt des Metallalkoxids M(OR)ₓ mit einem Halogengehalt > 200ppm als Verunreinigung 0.1 bis 10,0 Gew.-% ein- oder mehrkerniger halogenhaltiger Metallalkoxide enthält.

7. Verfahren gemäß wenigstens einem der Ansprüche 1 bis 6, **dadurch gekennzeichnet, dass** das Rohprodukt des Metallalkoxids M(OR)ₓ mit 4 bis 12 Gew.-% eines Alkohols ROH, bezogen auf die Gesamtmenge des rohen Alkoxids, gemischt wird.

8. Verfahren gemäß wenigstens einem der Ansprüche 1 bis 7, **dadurch gekennzeichnet, dass** die Menge an Ammoniak 0,1 bis 5,0 Gew.-%, bezogen auf die Gesamtmenge des rohen Alkoxids, beträgt.

9. Verbindungen M₂(OR)₉Hal, wobei R die in Anspruch 1 genannte Bedeutung habt, M für Ta oder Nb steht und 1 Hal ein 1 Halogen aus der Gruppe F, Cl, Br und I ist.

10. Verbindungen gemäß Anspruch 9, **dadurch gekennzeichnet, dass** Hal für Cl steht.

11. Verbindungen M₂(OR)₂Hal gemäß Anspruch 69, wobei R für einen C₁-C₅-Alklrest und Hal für Cl steht.

12. Verbindung nach Anspruch 11, **dadurch gekennzeichnet, dass** diese Verbindung Ta₂(OEt)₉Cl ist.

13. Gemische aus Verbindungen M₂(OR)₉Hal gemäß wenigstens einem der Ansprüche 9 bis 12 und M(OR)₅ mit mindestens 0,05 Gew.-% M₂(OR)₉Hal, wobei bei M(OR)₅ M für Ta oder Nb, und R unabhängig voneinander für gleiche oder verschiedene C₁-C₁₂-Alkylreste steht.

14. Gemische gemäß Anspruch 13, wobei R für einen C₁-C₅-Alkylrest und Hal für Cl steht.

15. Gemische gemäß Anspruch 13 oder 14 bestehend aus Ta₂(OEt)₉Cl und a(OEt)₅.

## Claims

1. Process for the preparation of highly pure metal alkoxides M(OR)ₓ, in which M represents Nb, Ta, Zr or Hf, x in the case where M = Nb or Ta represents 5 and in the case where M = Zr or Hf represents 4 and R independently of each other represents identical or different C₁-C₁₂-alkyl radicals, **characterized in that**
• a crude product of the metal alkoxide M(OR)ₓ with a halogen content > 200 ppm, which contains at least 0.05 wt.% of mono- or polynuclear halogen-containing metal alkoxides as an impurity,
• is mixed with a maximum of 30 wt.%, based on the total amount of the crude alkoxide, of an alcohol ROH, wherein R represents a C₁-C₁₂-alkyl radical and
• an excess, based on the amount of mono- or polynuclear halogen-containing metal alkoxides, of ammonia is subsequently or simultaneously metered in.

2. Process according to claim 1, **characterized in that** M represents Ta or Nb and the mono- or polynuclear halogen-containing metal alkoxide contained as an impurity is the compound M₂(OR)₉Hal, wherein Hal represents F, Cl, Br or I and R has the meaning given in claim 1.

3. Process according to claim 1, **characterized in that** M represents Zr or Hf and the mono- or polynuclear halogen-containing metal alkoxide(s) contained as an impurity is at least one compound Mₚ(OR)_{4p-q}Hal_{q}, wherein Hal represents F, Cl, Br or I, q represents 1, 2, 3 or 4, p represents 2, 3 or 4 and R has the meaning given in claim 1.

4. Process according to at least one of claims 1 to 3, **characterized in that** the radical R represents C₁-C₅-alkyl and Hal represents Cl.

5. Process according to at least one of claims 1 to 4, **characterized in that** the radical OR is an ethoxy group and M represents Ta.

6. Process according to at least one of claims 1 to 5, **characterized in that** the crude product of the metal alkoxide M(OR)ₓ with a halogen content > 200 ppm contains 0.1 to 10.0 wt.% of mono- or polynuclear halogen-containing metal alkoxides as an impurity.

7. Process according to at least one of claims 1 to 6, **characterized in that** the crude product of the metal alkoxide M(OR)ₓ is mixed with 4 to 12 wt.% of an alcohol ROH, based on the total amount of the crude alkoxide.

8. Process according to at least one of claims 1 to 7, **characterized in that** the amount of ammonia is 0.1 to 5.0 wt.%, based on the total amount of the crude alkoxide.

9. Compounds M₂(OR)₉Hal, wherein R has the meaning given in claim 1, M represents Ta or Nb and Hal is a halogen from the group F, Cl, Br and I.

10. Compounds according to claim 9, **characterized in that** Hal represents Cl.

11. Compounds M₂(OR)₉Hal according to claim 9, wherein R represents a C₁-C₅-alkyl radical and Hal represents Cl.

12. Compound according to claim 11, **characterized in that** this compound is Ta₂(OEt)₉Cl.

13. Mixtures of compounds M₂(OR)₉Hal according to at least one of claims 9 to 12 and M(OR)₅ with at least 0.05 wt.% of M₂(OR)₉Hal, wherein in M(OR)₅ independently of each other M represents Ta or Nb, and R represents identical or different C₁-C₁₂-alkyl radicals.

14. Mixtures according to claim 13, wherein R represents a C₁-C₅-alkyl radical and Hal represents Cl.

15. Mixtures according to claim 13 or 14 consisting of Ta₂(OEt)₉Cl and Ta(OEt)₅.

## Revendications

1. Procédé de production d'alcoxydes métalliques M(OR)ₓ de haute pureté, dans lesquels M désigne Nb, Ta, Zr ou Hf, x vaut 5 dans les cas où M = Nb ou Ta et vaut 4 dans les cas où M = Zr ou Hf, et les R, indépendamment les uns des autres, désignent des radicaux alkyles en C₁-C₁₂ identiques ou différents,
**caractérisé en ce que**
• un produit brut de l'alcoxyde métallique M(OR)ₓ présentant une teneur en halogène > 200 ppm qui contient comme impureté au moins 0,05 % en poids d'alcoxydes métalliques mono- ou multinucléaires contenant un halogène,
• est mélangé avec au maximum 30 % en poids, par rapport à la quantité totale d'alcoxyde brut, d'un alcool ROH, R désignant un radical alkyle en C₁-C₁₂, et
• ensuite ou simultanément, un excédent d'ammoniaque par rapport à la quantité d'alcoxydes métalliques mono- ou multinucléaires est ajouté.

2. Procédé selon la revendication 1, **caractérisé en ce que** M désigne Ta ou Nb et **en ce que** l'alcoxyde métallique mono- ou multinucléaire contenant un halogène contenu comme impureté est le composé M₂(OR)₉Hal, dans lequel Hal désigne F, Cl, Br ou I et R a la signification indiquée dans la revendication 1.

3. Procédé selon la revendication 1, **caractérisé en ce que** M désigne Zr ou Hf et **en ce que** le ou les alcoxyde(s) métallique(s) mono- ou multinucléaire(s) contenant un halogène contenu(s) comme impureté(s) est (sont) au moins un composé Mₚ(OR)_{4p-q}Hal_{q}, où Hal désigne F, Cl, Br ou I, q vaut 1, 2, 3 ou 4, p vaut 2, 3 ou 4 et R a la signification indiquée dans la revendication 1.

4. Procédé selon au moins l'une des revendications 1 à 3, **caractérisé en ce que** le radical R désigne un groupe alkyle en C₁-C₅ et Hal est Cl.

5. Procédé selon au moins l'une des revendications 1 à 4, **caractérisé en ce que** le radical OR désigne un groupe éthoxy et M est Ta.

6. Procédé selon au moins l'une des revendications 1 à 5, **caractérisé en ce que** le produit brut de l'alcoxyde métallique M(OR)ₓ présentant une teneur en halogène > 200 ppm contient comme impureté 0,1 à 10,0 % en poids d'un alcoxyde métallique mono- ou multinucléaire contenant un halogène.

7. Procédé selon au moins l'une des revendications 1 à 6, **caractérisé en ce que** le produit brut de l'alcoxyde métallique M(OR)ₓ est mélangé avec 4 à 12 % en poids d'un alcool ROH par rapport à la quantité totale d'alcoxyde brut.

8. Procédé selon au moins l'une des revendications 1 à 7, **caractérisé en ce que** la quantité d'ammoniaque est de 0,1 à 5,0 % en poids par rapport à la quantité totale d'alcoxyde brut.

9. Composés M₂(OR)₉Hal, dans lesquels R a la signification indiquée dans la revendication 1, M désigne Ta ou Nb et Hal désigne un halogène du groupe comprenant F, Cl, Br et I.

10. Composés selon la revendication 9, **caractérisés en ce que** Hal désigne Cl.

11. Composés M₂(OR)₉Hal selon la revendication 9, dans lesquels R désigne un radical alkyle en C₁-C₅ et Hal désigne Cl.

12. Composé selon la revendication 11, **caractérisé en ce que** ce composé est Ta₂(OEt)₉Cl.

13. Mélanges de composés M₂(OR)₉Hal selon au moins l'une des revendications 9 à 12 et de M(OR)₅ comportant au moins 0,05 % en poids de M₂(OR)₉Hal, où, dans le cas de M(OR)₅, M désigne Ta ou Nb et les R, indépendamment les uns des autres, désignent des radicaux alkyles en C₁-C₁₂ identiques ou différents.

14. Mélanges selon la revendication 13, dans lesquels R désigne un radical alkyle en C₁-C₅ et Hal désigne Cl.

15. Mélanges selon la revendication 13 ou 14, consistant en Ta₂(OEt)₉Cl et Ta(OEt)₅.
